# EUROPEAN PATENT APPLICATION

(11) **EP 2 387 946 A1**
(43) Date of publication of application: **23.11.2011**
(21) Application number: 11163084.4
(22) Date of filing: 19.04.2011
(51) Int. Cl.: A61B 8/00

(54) **Automatic balancing device and method using fluid**

(30) Priority: 18.05.2010 KR 20100046414
(71) Applicant: Samsung Medison Co., Ltd., Nam-myun Hongchun-gun Kangwon do 250-875 (KR)
(72) Inventor: Ko, Jae Yong, Seoul (KR); Woo, Kyeong Gu, Gyeonggi-do (KR)
(74) Representative: Schmid, Wolfgang

(57) **Abstract**

The present disclosure provides an automatic balancing device and method using fluid. The automatic balancing device (100) includes a fluid container (10) receiving a fluid (40) therein, a balance detector (20) detecting a balance of a main body (5) in association with the fluid (40), and a fluid regulator (30) adjusting movement of the fluid (40) received in the fluid container (10) in association with the balance detector (20). The automatic balancing device and method may prevent a medical device such as an ultrasonic diagnostic apparatus from falling over by shifting the center of gravity of the main body of the medical device through movement of the fluid.

## Description

### BACKGROUND

### 1. Technical Field

The present disclosure relates to an automatic balancing device and method using fluid and, more particularly, to an automatic balancing device and method, which uses a fluid to prevent a medical device from falling over by allowing rapid change of the center of gravity of the medical device when the medical device leans in one direction.

### 2. Description of the Related Art

Generally, an ultrasonic diagnostic apparatus refers to a non-invasive apparatus that irradiates an ultrasound signal from a surface of a patient body towards a target internal organ beneath the body surface and obtains an image of a monolayer or blood flow in soft tissue from information in the reflected ultrasound signal (ultrasound echo-signal).

The ultrasonic diagnostic apparatus has been widely used for diagnosis of the heart, the abdomen, the urinary organs, and in obstetrics and gynecology due to various merits thereof such as small size, low price, real-time image display, and high stability through elimination of radiation exposure, as compared with other image diagnostic systems, such as X-ray diagnostic systems, computerized tomography scanners (CT scanners), magnetic resonance imagers (MRIs), nuclear medicine diagnostic apparatuses, and the like.

It should be noted that the above description is provided for understanding of the background art and is not a description of a well-known conventional technique to which the present disclosure pertains.

In accordance with medical device regulations, when a medical device such as an ultrasonic diagnostic apparatus weighs 25 kg or more, the medical device must not fall over when pushed by a weight corresponding to 25% of the weight of the medical device. However, the current trend towards increasingly slim and lightweight medical devices makes it difficult for the medical device to satisfy this regulation. In particular, when the medical device such as an ultrasonic diagnostic apparatus includes wheels, it is difficult to adopt a separate fixing mechanism for the ultrasonic diagnostic apparatus.

### BRIEF SUMMARY

The present disclosure is directed to solving such problems of the related art, and aspects of the present disclosure provide an automatic balancing device and method, which use a fluid to prevent a medical device such as an ultrasonic diagnostic apparatus from falling over by allowing rapid change of the center of gravity of the medical device when the medical device tilts due to application of external force.

In accordance with one aspect, an automatic balancing device using fluid includes: a fluid container receiving a fluid therein; a balance detector detecting a balance of a main body in association with the fluid; and a fluid regulator adjusting movement of the fluid received in the fluid container in association with the balance detector.

The fluid container may include a first space and a second space divided from each other by the fluid to allow the fluid to move from one space to the other space by pressure difference therebetween.

The fluid regulator may include a pressure regulator connected to the fluid container and adjusting an internal pressure of the fluid container.

The fluid regulator may include a pressure reducer communicating at one side thereof with the first space and at the other side thereof with the second space to control pressures of the first and second spaces; a first on-off member communicating with the first space and adjusting the pressure of the first space by selectively opening or closing the first space; and a second on-off member communicating with the second space and adjusting the pressure of the second space by selectively opening or closing the second space.

The fluid regulator may include a first pressure detector disposed in the first space and detecting the pressure of the first space; a second pressure detector disposed in the second space and detecting the pressure of the second space; and a space pressure regulator adjusting the pressure of at least one of the first and second spaces in association with the first and second pressure detectors.

The main body may include an ultrasonic diagnostic apparatus.

In accordance with another aspect, an automatic balancing method using fluid includes: detecting a balanced state of a main body, to which a fluid container is provided; and returning the main body to the balanced state by moving a fluid in the fluid container based on pressure difference in the fluid container, if the main body is inclined to one side.

The returning the main body may include: opening one of first and second spaces in the fluid container maintained at sub-atmospheric pressure when the main body is inclined to the one of the first and second spaces; moving the fluid from the one of the first and second spaces to the other space based on pressure difference therebetween; adjusting a pressure of the other space to be the same as that of the one space by opening the other space after the fluid is moved to the one space; and adjusting the pressures of the first and second spaces to be lower than atmospheric pressure.

### BRIEF DESCRIPTION OF THE DRAWINGS

The above and other features and advantages of the present disclosure will become apparent from the following description of exemplary embodiments given in conjunction with the accompanying drawings, in which:
Fig. 1 is a schematic view of an ultrasonic diagnostic apparatus including an automatic balancing device using fluid according to one exemplary embodiment of the present disclosure;
Fig. 2 is a side section view of a fluid regulator of the automatic balancing device according to one exemplary embodiment of the present disclosure;
Fig. 3 is a block diagram of the fluid regulator of the automatic balancing device according to the one exemplary embodiment of the present disclosure;
Fig. 4 is a side section view of a fluid regulator of the automatic balancing device according to another exemplary embodiment of the present disclosure;
Fig. 5 is a block diagram of the fluid regulator of the automatic balancing device according to the other exemplary embodiment of the present disclosure;
Fig. 6 is a flowchart of an operation of the automatic balancing device using fluid according to one exemplary embodiment of the present disclosure; and
Fig. 7 is a flowchart of a process for maintaining a balanced state through movement of fluid in Fig. 6.

### DETAILED DESCRIPTION

Embodiments of the invention will now be described in detail with reference to the accompanying drawings. It should be noted that the drawings are not to precise scale and may be exaggerated in thickness of lines or size of components for descriptive convenience and clarity only. Furthermore, terms used herein are defined by taking functions of the invention into account and can be changed according to the custom or intention of users or operators. Therefore, definition of the terms should be made according to overall disclosures set forth herein.

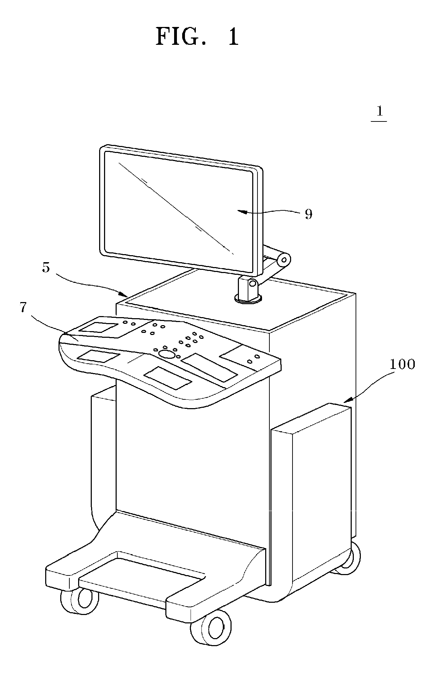

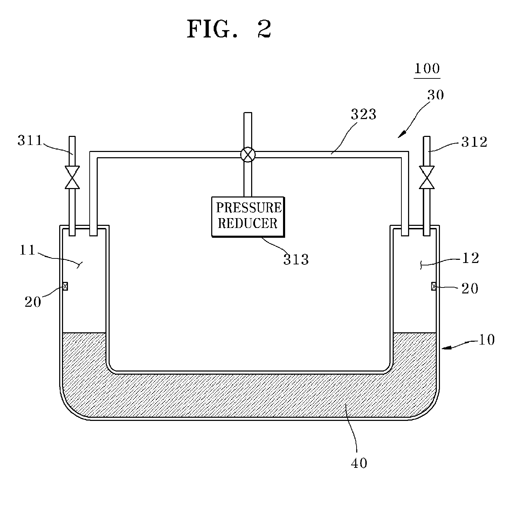

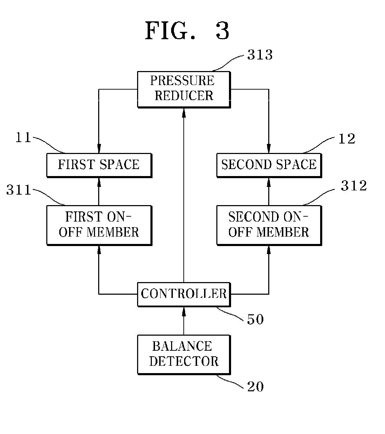

Fig. 1 is a schematic view of an ultrasonic diagnostic apparatus including an automatic balancing device using fluid according to an exemplary embodiment of the present disclosure, and Figs. 2 and 3 are a side section view and a block diagram of a fluid regulator of the automatic balancing device according to one exemplary embodiment of the present disclosure.

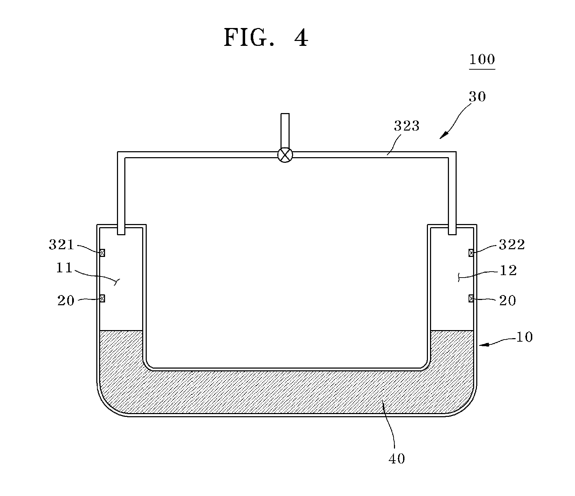

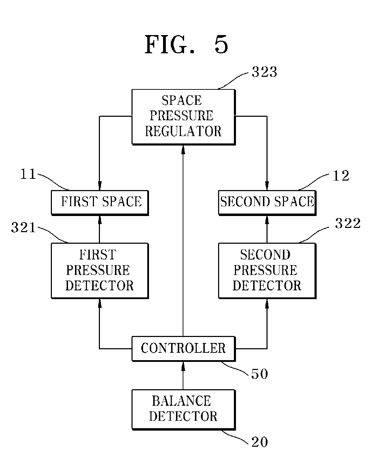

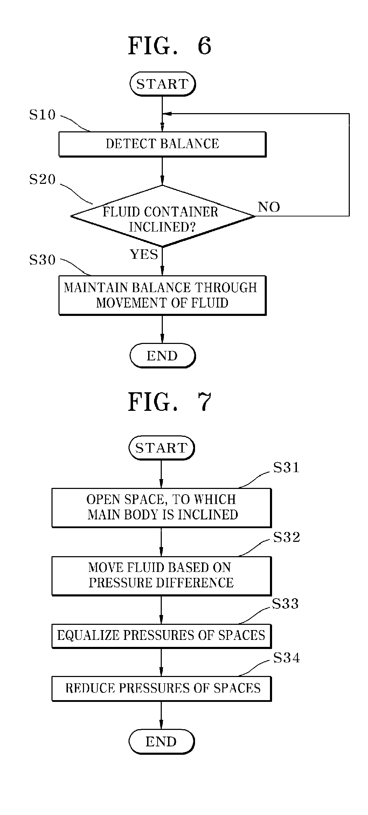

Figs. 4 and 5 are a side section view and a block diagram of a fluid regulator of the automatic balancing device according to another exemplary embodiment of the present disclosure, Fig. 6 is a flowchart of an operation of an automatic balancing device using fluid according to one exemplary embodiment of the present disclosure, and Fig. 7 is a flowchart of a process for maintaining a balanced state through movement of fluid in Fig. 6.

Referring to Fig. 1, an automatic balancing device 100 according to one exemplary embodiment is provided to a main body 5 of an ultrasonic diagnostic apparatus 1. The main body 5 of the apparatus 1 includes a beam former (not shown) for transmit-focusing ultrasound signals transmitted through a probe and receive-focusing ultrasound signals received through the probe, a data creator (not shown) for creating frame data based on the signals output from the beam former, a processor (not shown) for generating a two-dimensional or three-dimensional image of a target based on the frame data, and a storage (not shown) for storing data.

The main body 5 is further provided with a control panel 7 for operating the ultrasonic diagnostic apparatus 1 or selecting functions thereof, and with a display unit 9 for displaying state information of the probe and the like.

The automatic balancing device 100 may be mounted on or received inside the main body 5 of the apparatus 1. The automatic balancing device 100 may be mounted on other apparatuses including medical devices, which require weight balancing.

Referring to Figs. 2 and 4, the automatic balancing device 100 includes a fluid container 10, balance detectors 20, and a fluid regulator 30.

The fluid container 10 receives a fluid 40 which serves to maintain a balance of the main body 5. As shown in Fig. 1, the fluid container 10 may be mounted on or received inside the main body 5. According to one embodiment, the fluid container 10, upper sides of which are separated from each other and lower sides of which communicate with each other, has a U-shaped cross-section. The fluid container 10 receives the fluid 40. The fluid 40 is collected in a lower portion of the fluid container 10 by gravity. In this embodiment, the fluid container 10 includes a first space 11 and a second space 12 divided from each other by the fluid 40. The first and second spaces 11, 12 are held at sub-atmospheric pressure, for example, in a vacuum.

The balance detectors 20 detect the balance of the main body 5 in association with the fluid 40. In other words, when the main body 5 is inclined towards one side and becomes unbalanced, the fluid 40 in the fluid container 10 is moved in association with the tilting behavior of the main body 5 and the balancing detectors 20 detect such movement of the fluid 40. In this embodiment, the balancing detectors 20 are mounted inside the fluid container 10. The balancing detectors 20 are provided to the first and second spaces 11, 12, respectively. Accordingly, if the fluid 40 is moved to one of the first and second spaces 11, 12 by inclination of the fluid container 10, an associated balance detector 20 will be brought into contact with the fluid 40 and detect the inclination of the fluid container 10.

The fluid regulator 30 adjusts movement of the fluid 40 received in the fluid container 10 in association with the balance detectors 20. In this embodiment, the fluid regulator 30 includes a pressure regulator which adjusts an internal pressure of the fluid container 10. The pressure regulator adjusts pressures of the first and second spaces 11, 12 to move the fluid 40. The fluid container 10 and ultimately the main body 5 of Fig. 1 are then balanced through movement of the fluid 40 by the pressure regulator.

Referring to Figs. 2 and 3, a fluid adjustor 30 according to one exemplary embodiment of the present disclosure includes a pressure reducer 313, a first on-off member 311, and a second on-off member 312.

The pressure reducer 313 communicates at one side thereof with the first space 11 and at the other side thereof with the second space 12 to control the internal pressures of the first and second spaces 11, 12. Specifically, the pressure reducer 313 adjusts the pressures of the first and second spaces 11, 12 to be lower than atmospheric pressure.

The first on-off member 311 communicates with the first space 11 and selectively opens or closes the first space 11 to adjust the pressure of the first space 11. Since atmospheric pressure is higher than the pressure of the first space 11, air flows into the first space 11 and the fluid 40 is then moved to the second space 12 when the first on-off member 311 is opened.

The second on-off member 312 communicates with the second space 12 and selectively opens or closes the second space 12 to adjust the pressure of the second space 12. Since atmospheric pressure is higher than the pressure of the second space 12, air flows into the second space 12 and the fluid 40 is then moved to the first space 11 when the second on-off member 312 is opened.

A controller 50 controls the first on-off member 311, second on-off member 312 and pressure reducer 313 in association with the balance detectors 20. Thus, when the fluid container 10 is inclined towards a right side in Fig. 2, the associated balance detector 20 sends information about inclination of the fluid container 10 to the controller 50. Upon receiving a signal from the balance detector 20, the controller 50 opens the second on-off member 312 so that air flows into the second space 12 therethrough. When the air is introduced into the second space 12, the pressure of the second space 12 rises above the pressure of the first space 11, so that the fluid 40 is moved to the first space 11 by pressure difference between the first and second spaces. As the fluid 40 flows into the first space 11 by the pressure difference between the first and second spaces 11, 12, the center of gravity of the balancing device shifts to the first space 11, thereby preventing the main body from falling over. When the fluid container 10 is balanced, the controller 50 controls the pressure reducer 313 such that the pressures of the first and second spaces 11, 12 become lower than external pressure.

On the other hand, the balance detectors 20 may detect the balance of the fluid container 10 in a variety of ways depending on variation of inclination of the fluid container 10 and installation locations. For example, when the balance detectors 20 are disposed to contact the fluid 40 with the fluid container 10 balanced, inclination of the fluid container 10 to the right side causes the fluid 40 to flow into the second space 12 and moves away from the balance detector 20 in the first space 11. At this time, the balance detector 20 in the first space 11 sends a signal to the controller 50, which in turn recognizes, through movement of the fluid 40 into the second space 12, that the fluid container 10 is inclined. In another example, when the balance detectors 20 are disposed to be separated from the fluid 40 and located at upper portions of the first and second spaces 11, 12 with the fluid container 10 balanced, inclination of the fluid container 10 to the right side causes the fluid 40 to be brought into contact with the balance detector 20 in the second space 12. At this time, the balance detector 20 in the second space 12 sends a signal to the controller 50, which in turn recognizes, through movement of the fluid 40 into the second space 12, that the fluid container 10 is inclined.

Referring to Figs. 4 and 5, a fluid regulator 30 according to a second exemplary embodiment of the present disclosure includes a first pressure detector 321, a second pressure detector 322 and a space pressure regulator 323.

The first pressure detector 321 is disposed inside the first space 11 and detects an internal pressure of the first space 11, and the second pressure detector 322 is disposed inside the second space 12 and detects an internal pressure of the second space 12.

The space pressure regulator 323 adjusts the pressure of one of the first and second spaces 11, 12 in association with the first and second pressure detectors 321, 322.

The controller 50 controls the first pressure detector 321, second pressure detector 322, and space pressure regulator 323 in association with the balance detectors 20.

For example, when the fluid container 10 is inclined to the right side in Fig. 4, the balance detector 20 sends information about inclination of the fluid container 10 to the controller 50. At this time, as the pressures of the first and second containers 11, 12 vary due to movement of the fluid 40, the first and second pressure detectors 321, 322 send pressure values of the first and second spaces 11, 12 to the controller 50. The controller 50 controls the space pressure regulator 323 in response to a signal from the balance detector 20 and balances the fluid container 10 by moving the fluid 40 to the first space 11 through adjustment of the pressure of the second space 12. At this time, the controller 50 controls operation of the space pressure regulator 323 until the first and second spaces 11, 12 have the same pressure.

Next, an automatic balancing method of the automatic balancing device using fluid according to the embodiment will be described.

With the fluid container 10 attached to the main body 5 of the medical device such as an ultrasonic diagnostic apparatus, the balancing detectors 20 detect a balance of the main body in S10. Specifically, the balance detectors 20 are received in the fluid container 10 and send information about inclination of the main body 5 to the controller 50 through detection of the inclination of the main body 5 when the fluid 40 is moved.

If the balance detectors 20 detect movement of the fluid 40 in S20, the controller 50 controls the fluid regulator 30, which in turn forces the fluid 40 to move, in 530.

Since movement of the fluid 40 causes the center of gravity of the fluid container 10 to shift, the main body 5 may ultimately return from the inclined state to a balanced state.

A process of maintaining the balance of the main body 5 using the fluid regulator 30 will be briefly described.

First, when the fluid container 10 is inclined to one of the first and second spaces 11, 12 in the fluid container 10, which are divided from each other by the fluid 40 and are maintained at sub-atmospheric pressure, the controller 50 opens the space, to which the fluid container 10 is inclined, in 531.

Specifically, when the fluid container 10 is inclined to the right side, the controller 50 opens the second on-off member 312 and allows external air to flow into the second space 12 through the second on-off member 312. Although the first and second spaces 11, 12 are maintained in a vacuum, the flow of air into the second space causes an increase in pressure of the second space 12 above the pressure of the first space 11.

Then, pressure difference between the first and second spaces 11, 12 forces the fluid 40 to move into the first space 11, which has a lower pressure than the second space 12, in S32.

Movement of the fluid 40 resulting from the pressure difference shifts the center of gravity of the main body 5 including the fluid container 10, so that the main body 5 returns from the inclined state to the balanced state.

When the fluid container 10 returns to the balanced state through movement of the fluid 40 to the first space 11, the controller 50 additionally opens the first on-off member 311 in S33. Then, air is introduced into the first and second spaces 11, 12, so that the first and second spaces 11, 12 have the same pressure.

The controller 50 may detect, using a separate pressure detector, whether the first and second spaces 11, 12 have the same pressure. Alternatively, the controller 50 may allow standby time until the first and second spaces 11, 12 have the same pressure.

When the first and second spaces 11, 12 have the same pressure, the pressures of the first and second spaces 11, 12 are lowered in S34.

The controller 50 controls the pressure reducer 313 to discharge air from the first and second spaces 11, 12 such that the first and second spaces 11, 12 are maintained at sub-atmospheric pressure.

As such, according to the embodiments of the present disclosure, the automatic balancing device and method may prevent a medical device such as an ultrasonic diagnostic apparatus from falling over by shifting the center of gravity of a main body of the medical device through movement of fluid.

Further, according to the embodiments, the automatic balancing device and method allow rapid and accurate movement of the fluid based on pressure difference.

Although the present disclosure has been described with reference to the embodiments shown in the drawings, it should be understood by those skilled in the art that these embodiments are given by way of illustration only, and that various modifications, variations, and alternations can be made without departing from the spirit and scope of the present disclosure. Further, although the ultrasonic diagnostic apparatus is illustrated herein, the present disclosure is obviously applicable to other medical devices and may be realized in various ways. Therefore, the scope of the present disclosure should be limited only by the accompanying claims and equivalents thereof.

## Claims

1. An automatic balancing device (100) using fluid, **characterized by** comprising:
a fluid container (10) receiving a fluid (40) therein;
a balance detector (20) detecting a balance of a main body (5) in association with the fluid (40); and
a fluid regulator (30) adjusting movement of the fluid (40) received in the fluid container (10) in association with the balance detector (20).

2. The device (100) according to claim 1, **characterized in that** the fluid container (10) comprises a first space (11) and a second space (12) divided from each other by the fluid (40) to allow the fluid to move from one space to the other space by pressure difference therebetween.

3. The device (100) according to claim 2, **characterized in that** the fluid regulator (30) comprises a pressure regulator connected to the fluid container (10) and adjusting an internal pressure of the fluid container (10).

4. The device (100) according to claim 3, **characterized in that** the fluid regulator (30) comprises:
a pressure reducer (313) communicating at one side thereof with the first space (11) and at the other side thereof with the second space (12) to control pressures of the first and second spaces (11, 12);
a first on-off member (311) communicating with the first space (11) and adjusting the pressure of the first space (11) by selectively opening or closing the first space (11); and
a second on-off member (312) communicating with the second space (12) and
adjusting the pressure of the second space (12) by selectively opening or closing the second space (12).

5. The device (100) according to claim 3, **characterized in that** the fluid regulator (30) comprises:
a first pressure detector (321) disposed in the first space (11) and detecting the pressure of the first space (11);
a second pressure detector (322) disposed in the second space (12) and detecting the pressure of the second space (12); and
a space pressure regulator (323) adjusting the pressure of at least one of the first and second spaces (11, 12) in association with the first and second pressure detectors (321, 322).

6. The device (100) according to claim 1, **characterized in that** the main body (5) comprises an ultrasonic diagnostic apparatus.

7. An automatic balancing method using fluid, **characterized by** comprising:
detecting a balanced state of a main body, to which a fluid container is provided; and
returning the main body to the balanced state by moving a fluid in the fluid container based on pressure difference in the fluid container, if the main body is inclined to one side.

8. The method according to claim 7, **characterized in that** the returning of the main body comprises:
opening one of first and second spaces of the fluid container when the main body is inclined to the one of the first and second spaces divided by the fluid and maintained at sub-atmospheric pressure;
moving the fluid from the one of the first and second spaces to the other space based on pressure difference therebetween;
adjusting a pressure of the other space to be the same as that of the one space by opening the other space after the fluid is moved to the one space; and
adjusting the pressures of the first and second spaces to be lower than atmospheric pressure.
